# EUROPÄISCHE PATENTSCHRIFT

(11) **EP 1 004 565 B1**
(45) Veröffentlichungstag und Bekanntmachung des Hinweises auf die Patenterteilung: **23.10.2002**
(21) Anmeldenummer: 00101566.8
(22) Anmeldetag: 14.08.1997
(51) Int. Cl.: C07C 43/15, C07C 41/28

(54) **Herstellung eines 1-Alkoxy-2-methyl-1, 3-butadiens**
Preparation of 1-alkoxy-2-methyl-1,3-butadienes
Préparation de 1,3-butadiènes 1-alkoxy-2-méthyl

(30) Priorität: 19.08.1996 EP 96113248
(43) Veröffentlichungstag der Anmeldung: 31.05.2000
(62) Teilanmeldung aus: 97114056.1
(73) Patentinhaber: F. HOFFMANN-LA ROCHE AG, 4070 Basel (CH)
(72) Erfinder: Nösberger, Paul, 4127 Birsfelden (CH)

(56) Entgegenhaltungen:
- S. M. MAKIN: "The enol synthesis of polyenes" PURE AND APPLIED CHEMISTRY, Bd. 47, Nr. 2/3, 1976, Seiten 173-181, XP002055722
- CHEMICAL ABSTRACTS, vol. 41, no. 19, 10. Oktober 1947 (1947-10-10) Columbus, Ohio, US; W. FLAIG: "New transformation of crotonaldehyde" Spalte 6189; XP002132940 & REICHSAMT WIRTSCHAFTSAUSBAU, CHEM. BER. PRÜF. NR. 093 (PB52020),1942, Seiten 1073-1108,
- L. CERVENY ET AL: "Catalytic splitting of acetals to unsaturated ethers" J. CHEM. TECH. BIOTECHNOL., Bd. 58, Nr. 3, 1993, Seiten 211-214, XP000404018

## Beschreibung

Die vorliegende Erfindung betrifft ein neues Verfahren zur Herstellung eines 1-Alkoxy-2-methyl-1,3-butadiens durch katalytische Dealkoxylierung eines 1,1,3-Trialkoxy-2-methyl-butans in der Gasphase. Das Produkt dieses Verfahrens eignet sich beispielsweise zur Herstellung eines γ-Halogentiglinaldehyds, welcher seinerseits zur Herstellung von γ-Acetoxy-tiglinaldehyd oder von gewissen C₅-Wittigaldehydhalogeniden verwendet werden kann. Diese Produkte sind bekannte, wichtige Zwischenprodukte für die Herstellung von verschiedenen Apocarotinalen und Diapocarotinalen sowie von Vitamin A.

Die europäische Patentanmeldung Nr. 97114056.1 [Veröffentlichungsnr. (EP) 0825168 A2], zu welcher die vorliegende Anmeldung eine Teilanmeldung ist, beschreibt und beansprucht ein Verfahren zur Herstellung von γ-Chlor- oder γ-Bromtiglinaldehyd der allgemeinen Formel

HalH₂C-CH=C(CH₃)-CHO I

worin Hal Chlor oder Brom bedeutet.
Dieses Verfahren ist dadurch gekennzeichnet, dass man ein 1-Alkoxy-2-methyl-1,3-butadie der allgemeinen Formel

H₂C=CH-C(CH₃)=CH-OR¹ II

worin R¹ C₁₋₄-Alkyl bedeutet,
mittels eines Halogenierungsmittels, welches aus einem Alkalimetallhypochlorit, einem Alkalimetallhypobromit, einem Erdalkalimetallhypochlorit, einem Erdalkalimetallhypobromit, tert.Butylhypochlorit, N-Bromacetamid, 1,3-Dichlor-5,5-dimethyl-hydantoin und 1,3-Dibrom-5,5-dimethyl-hydantoin ausgewählt ist, in einem Alkohol R²OH, worin R² C₁₋₄-Alkyl bedeutet, haloalkoxyliert, und das so erhaltene γ-Halogentiglinaldehyd-dialkylacetal der allgemeinen Formel

HalH₂C-CH=C(CH₃)-CH(OR¹)(OR²) III

worin Hal, R¹ und R² die oben angegebenen Bedeutungen besitzen, zum gewünschten γ-Chlor- bzw. γ-Bromtiglinaldehyd der Formel I hydrolysiert. In dieser Definition sind unter dem Ausdruck "C₁₋₄-Alkyl" sowohl geradkettige als auch (ab C₃) verzweigte Alkylgruppen zu verstehen. Vorzugsweise ist die Alkylgruppe allerdings Methyl oder Ethyl, was sowohl für R¹ als auch für R² gilt.

Weitere Details über die Herstellung von γ-Chlor- oder γ-Bromtiglinaldehyd sind in der oben erwähnten EP 0825168 A2 zu finden.

Die als Ausgangsmaterialien in dem obigen Verfahren verwendeten 1-Alkoxy-2-methyl-1,3-butadiene der Formel II sind zum Teil bekannte Verbindungen; die restlichen (neuen) können aus bekannten Ausgangsmaterialien nach an sich bekannten Methoden hergestellt werden.

So ist beispielsweise das 1-Ethoxy-2-methyl-1,3-butadien (der Formel II, worin R¹ Ethyl bedeutet) aus der Literatur seit langem bekannt [siehe u.a. J.A.C.S. 91, 3281 ff. (1969), Bull. Soc. Chim. Fr. 1963, 1646 ff., sowie J. Gen. Chem. USSR 29, 3649 ff. (1959)] und wurde jeweils durch zweimalige Abspaltung von Ethanol aus 1,1,3-Triethoxy-2-methyl-butan hergestellt. Das Butanderivat seinerseits kann durch eine seit langem bekannte Enoletherkondensation (siehe US-Patentschrift 2.165.962) aus den beiden gut zugänglichen Ausgangsmaterialien Acetaldehyd-diethylacetal und Ethyl-(1-propenyl)-ether hergestellt werden [siehe zudem J.A.C.S. 71, 3468 ff. (1949) sowie J. Gen. Chem. USSR 29, 3641 ff. (1959)]. Hierbei erwärmt man bei etwa 35°C etwa 2 bis 3 Aequivalente des Acetals pro Aequivalent Ethylpropenylether bis zu etwa 2 Stunden in Gegenwart von etwa 0,2 Molprozent Bortrifluoridetherat als Katalysator ohne Lösungsmittel und erhält das gewünschte Butanderivat in etwa 66%iger Ausbeute. Die anschliessende zweimalige Abspaltung von Ethanol aus dem 1,1,3-Triethoxy-2-methyl-butan kann gemäss dem einschlägigen Stand der Technik auf zwei verschiedenen Wegen realisiert werden:
(i) durch Abspaltung in der Flüssigphase, indem man das 1,1,3-Triethoxy-2-methyl-butan in Isochinolin enthaltend eine katalytische Menge p-Toluolsulfonsäure bei etwa 220°C zutropft und das in der Folge gebildete 1-Ethoxy-2-methyl-1,3-butadien abdestilliert. Die Ausbeuten sind bei dieser in Bull. Soc. Chim. Fr. 1963, 1646 ff. beschriebenen Methode allerdings mittelmässig (etwa 40-50%); oder
(ii) durch Abspaltung in der Gasphase bei 300-350°C unter Vakuum an einem sauren Katalysator, z.B. Mononatriumphosphat [J. Gen. Chem. USSR 29, 3649 ff. (1959)].

Auch das 1-Methoxy-2-methyl-1,3-butadien (der Formel II, worin R¹ Methyl bedeutet) ist aus der Literatur [Japanische Patentpublikation (Kokai) 50891/1989] bekannt. Es kann beispielsweise analog der oben beschriebenen Herstellung von 1-Ethoxy-2-methyl-1,3-butadien ausgehend von Acetaldehyd-dimethylacetal und Methyl-(1-propenyl)-ether über das 1,1,3-Trimethoxy-2-methyl-butan hergestellt werden.

Die restlichen 1-Alkoxy-2-methyl-1,3-butadiene der Formel II sind zum Teil bekannte Verbindungen und können analog den obenerwähnten Verbindungen hergestellt werden. Das nachfolgende Reaktionsschema stellt eine Zusammenfassung des oben näher erläuterten mehrstufigen Verfahrens dar, gemäss welchem alle 1-Alkoxy-2-methyl-1,3-butadiene der Formel II hergestellt werden können:

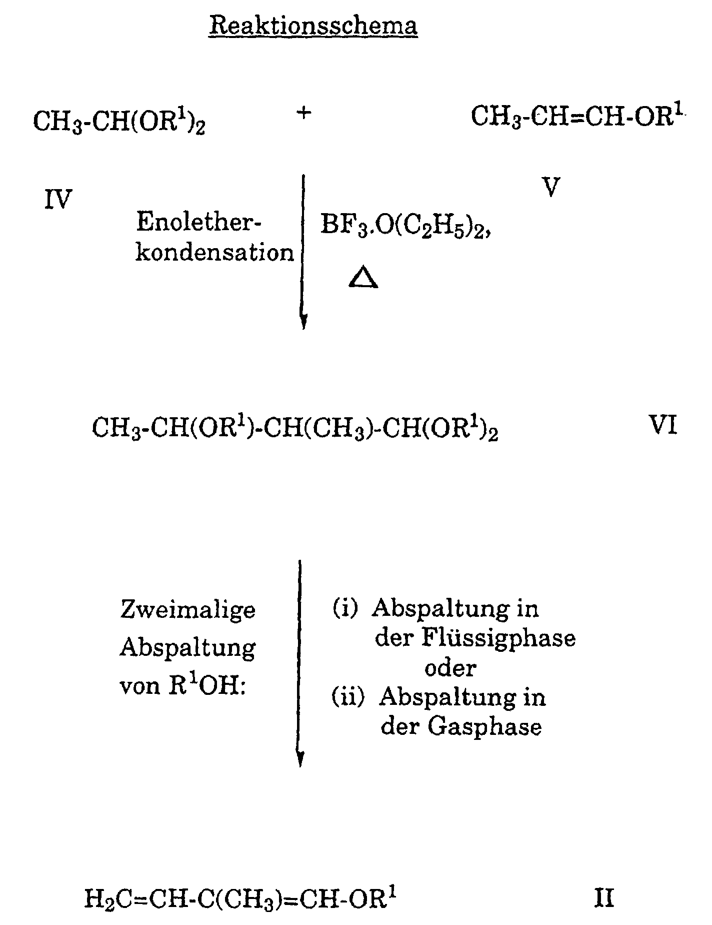

In diesem Reaktionsschema besitzt R¹ die oben angegebene Bedeutung. Das Zwischenprodukt der Formel VI und das Produkt der Formel II können auf an sich bekannte Weise isoliert und gereinigt werden.

Uebersichtsartikel zur Herstellung der 1-Alkoxy-2-methyl-1,3-butadiene befinden sich in Russian Chem. Rev. 38, 237 ff. (1969) und in Pure & Appl. Chem. 47, 173 ff. (1976); für weitere Literatur über deren Herstellung durch Gasphasenkatalyse wird auf Lieb. Ann. Chem. 568, 1 ff. (1950), Can. J. Res. B 28, 689 ff. (1950), ibid. B 25, 118 ff. (1947) sowie Chem. Ber. 77, 108 ff. (1944) verwiesen.

Die Abspaltung des Alkohols R¹OH (Dealkoxylierung) von den Trialkoxybutanen der Formel VI in der Gasphase [Methode (ii)] bei erhöhter Temperatur an einem fixierten, festen Katalysator ("Festbettkatalysator") ist bei einem technischen Prozess viel attraktiver als eine Abspaltung in der Flüssigphase [Methode (i)], da die Methode (ii) u.a. kein Lösungsmittel benötigt und eine einfache Reaktionsführung und Aufarbeitung aufweist. Daher ist die in der Gasphase erfolgende Dealkoxylierung bevorzugt. Man kann im allgemeinen als Katalysatoren ähnliche verwenden, die zur Dealkoxylierung von unsubstituierten Acetalen eingesetzt werden. Zudem wird eine derartige Dealkoxylierung zweckmässigerweise bei Temperaturen zwischen 300°C und 350°C durchgeführt.

Es wurde bei der Untersuchung der Dealkoxylierung nun gefunden, dass sich ein bisher zu diesem Zweck noch nie verwendeter Typ von Katalysator besonders gut eignet. Bei diesem Katalysator handelt es sich um schwach saures Aluminiumsilikat. Aluminiumsilikat kommt bekanntlich als Gemisch von Aluminiumoxid (Al₂O₃) und Silika (SiO₂) vor, wobei das Mengenverhältnis Al₂O₃:SiO₂ über einen breiten Bereich variieren kann. Der schwach saure Aluminiumsilikat-Katalysator, welcher sich zur Katalyse der hier beschriebenen Dealkoxylierung eignet, weist eine mittlere spezifische Oberfläche auf, und zwar eine im Bereich von 5 bis 50 m²/g. Darüber hinaus befindet sich der Aluminiumoxid-Anteil des Aluminiumsilikat-Katalysators vorzugsweise in der α-Form.

Das erfindungsgemässe Verfahren zur katalytischen Dealkoxylierung eines 1,1,3-Trialkoxy-2-methyl-butans der oben angegebenen und erläuterten allgemeinen Formel VI zum entsprechenden 1-Alkoxy-2-methyl-1,3-butadien der ebenfalls oben angegebenen und erläuterten allgemeinen Formel II in der Gasphase ist dadurch gekennzeichnet, dass man als Katalysator schwach saures Aluminiumsilikat spezifischer Oberfläche im Bereich von 5 bis 50 m²/g verwendet.

Unter Verwendung eines solchen Katalysators mit einer spezifischen Oberfläche im Bereich von 5 bis 50 m²/g und darüber hinaus eines, dessen Aluminiumoxid-Anteil in der α-Form vorliegt, kann erfahrungsgemäss eine Abspaltungsselektivität von etwa 90% bei einem Umsatz von etwa 90% erreicht werden.

Das obige Verfahren wird zweckmässigerweise analog den bekannten Dealkoxylierungen in der Gasphase durchgeführt, wobei das kennzeichnende Merkmal der erfindungsgemässen Dealkoxylierung in der Verwendung des Aluminiumsilikat-Katalysators besteht. Es gelten für das erfindungsgemässe Dealkoxylierungsverfahren zweckmässigerweise Reaktionstemperaturen im Bereich von 250°C bis 400°C, vorzugsweise Temperaturen von 300°C bis 350°C, und zweckmässigerweise Drücke von 1 kPa bis 200 kPa. Vorzugsweise wird das Dealkoxylierungsverfahren bei Atmosphärendruck durchgeführt.

Zweckmässigerweise erfolgt die Dealkoxylierung in einem Festbettreaktor, wobei das Edukt 1,1,3-Trialkoxy-2-methyl-butan, gegebenenfalls mit einem Inertgas verdünnt, kontinuierlich über den Aluminiumsilikat-Katalysator als Festbett in einer Reaktionssäule geleitet wird und das resultierende entsprechende 1-Alkoxy-2-methyl-1,3-butadien am Ende der Reaktionssäule entnommen wird. Die Methodik ist an sich bekannt, was ebenfalls für die dazu benötigten Apparaturen gilt. Als Inertgas kann beispielsweise Stickstoff oder Kohlendioxid verwendet werden. Die Verweilzeiten am Katalysatorbett liegen zweckmässigerweise im Bereich von 0,1 bis 10 Sekunden, vorzugsweise im Bereich von 0,5 bis 5 Sekunden. Durch Einstellung der Reaktionstemperatur, Verweilzeit und weiterer Reaktionsparameter kann der Umsatz an Edukt optimiert werden, damit eine möglichst hohe Ausbeute an gewünschtem Produkt erzielt und möglichst wenig Nebenprodukte gebildet werden.

Wie eingangs erwähnt, kann der unter Verwendung des erfindungsgemäss hergestellten 1-Alkoxy-2-methyl-1,3-butadiens herstellbare γ-Chlor- oder γ-Bromtiglinaldehyd der Formel I zur Herstellung von verschiedenen weiteren Zwischenprodukten verwendet werden, welche ihrerseits schliesslich zur Herstellung von Apocarotinalen, Diapocarotinalen sowie Vitamin A von Nutzen sind. Die diesbezüglichen weiteren Angaben sind in der oben erwähnten EP 0825168A2 zu finden.

Die Erfindung wird anhand der nachfolgenden Beispiele veranschaulicht.

### Herstellung des 1-Alkoxy-2-methvl-1,3-butadiens (Verbindung der Formel II)

### Beispiel 1

### 1-Methoxy-2 -methyl-1,3-butadien [zwei Stufen a) und b)]

### a) 1,1,3-Trimethoxy-2-methyl-butan

In einem mit Magnetrührer, Thermometer und Tropftrichter ausgestatteten 2 l-Zweihalsrundkolben wurden unter Argon 1035 g (11 Mol) Acetaldehyd-dimethylacetal [Reinheit nach Gaschromatographie (GC): 96,5%] und 3,4 g (3 ml, 24 mMol, 0,22 Mol%) Bortrifluoridetherat vorgelegt. Zum Gemisch wurden innert 2 Stunden bei zwischen 30 und 40°C unter gelegentlichem Kühlen (die Reaktion war exotherm) 530 g (7,35 Mol) Methyl-(1-propenyl)-ether zugetropft. Nach beendeter Zugabe wurde noch 30 Minuten bei 30°C weitergerührt, dann auf Raumtemperatur abgekühlt. Man gab 3 ml Triethylamin zu, rührte 15 Minuten und nutschte ab. Der Ueberschuss an Acetaldehyd-dimethylacetal wurde nun an einer Raschig-Kolonne (50 x 3 cm) bei Normaldruck abdestilliert, und der Rückstand an der gleichen Kolonne fraktioniert. Dies ergab bei einem Druck von 50 mbar eine 1. Fraktion (Sdp. 81,5-83,5°C), bestehend aus 742 g (60%ige Ausbeute) 1,1,3-Trimethoxy-2-methyl-butan (Gehalt nach GC: 96,4%) als farblose Flüssigkeit, und eine 2. Fraktion (Sdp. 83,5-85°C), bestehend aus 87,5 g (6,3%ige Ausbeute) 1,1,3-Trimethoxy-2-methyl-butan (Gehalt nach GC: 86%), ebenfalls als farblose Flüssigkeit, d.h. eine totale Ausbeute von 66,3% des gewünschten Produktes.

### b) 1-Methoxy-2-methyl-1,3-butadien

### (i) (1. Variante)

Als Reaktor wurde ein elektrisch geheiztes Stahlrohr (Länge 60 cm, Durchmesser 2,7 cm, Wandtemperatur 300°C) verwendet. Das Reaktionsrohr war unten mit Keramikkugeln (10 cm hoch, Durchmesser 6 mm) gefüllt. Darüber befanden sich 100 ml Aluminiumsilikat-Katalysatorträger mit einer spezifischen Oberfläche von 15 m²/g. Der obere Teil des Reaktors wurde mit Keramikkugeln aufgefüllt. Dem Reaktor wurden kontinuierlich 60 ml/h 1,1,3-Trimethoxy-2-methyl-butan und 20 l/h Stickstoff zugeführt. Die Reaktionsprodukte wurden in einem auf 20°C gekühlten Kondensator mit eingebauter Fritte (Nebelabscheidung) aus dem Stickstoffstrom kondensiert, flüssig entnommen und gaschromatographisch analysiert. Der Umsatz an 1,1,3-Trimethoxy-2-methyl-butan betrug 88%, die Selektivität zu 1-Methoxy-2-methyl-1,3-butadien 90%. Nach Abtrennung des gebildeten Methanols durch Extraktion mit alkalischem Wasser (pH >8) wurde die organische Phase durch Rektifikation aufgetrennt. Man erhielt 1-Methoxy-2-methyl-1,3-butadien mit einer Reinheit von etwa 99%.

### (ii) (2. Variante)

Die Wandtemperatur des Reaktors wurde auf 250°C eingestellt. Alle anderen Bedingungen waren gleich wie in Beispiel b)(i). Der Umsatz an 1,1,3-Trimethoxy-2-methyl-butan betrug 41%, die Selektivität zu 1-Methoxy-2-methyl-1,3-butadien 90%.

### (iii) (3. Variante)

Die Wandtemperatur des Reaktors wurde auf 325°C eingestellt. Alle anderen Bedingungen waren gleich wie in Beispiel b)(i). Der Umsatz an 1,1,3-Trimethoxy-2-methyl-butan betrug 97,5%, die Selektivität zu 1-Methoxy-2-methyl-1,3-butadien 86%.

### Beispiel 2

### 1 -Ethoxy-2-methyl-1,3-butadien [zwei Stufen a) und b)]

### a) 1,1,3-Triethoxy-2-methyl-butan

In einem mit Magnetrüher, Thermometer und Tropftrichter ausgestatteten 2 l-Zweihalsrundkolben wurden unter Argon 900 g (7,4 Mol) Acetaldehyd-diethylacetal (Reinheit nach GC: 97%) und 0,85 g (0,75 ml, 6 mMol, 0,25Mol%) Bortrifluoridetherat vorgelegt. Unter gelegentlicher Eiskühlung wurden bei einer Temperatur von etwa 35°C, höchstens aber bei 40°C, 220 g (2,5 Mol) Ethyl-(1-propenyl)-ether innert etwa 30 Minuten zugetropft. Nach beendeter Zugabe wurde noch 30 Minuten bei Raumtemperatur nachgerührt. Dann gab man 4 g festes, pulverisiertes Natriumcarbonat zu und rührte 2,5 Stunden bei Raumtemperatur nach. Dann wurde abgenutscht und der Ueberschuss an Acetaldehyd-diethylacetal an einer Raschig-Kolonne (30 x 2,5 cm) bei 100 mbar abdestilliert. Bei einem Siedepunkt von 40-43°C wurde auf diese Weise etwa 1 l Acetaldehyd-diethylacetal (Gehalt nach GC: 91%) zurückerhalten. Der Rückstand wurde nun bei einem Druck von 12-13 mbar an der gleichen Kolonne fraktioniert. Dies ergab 344,5 g (66,2%ige Ausbeute) 1,1,3-Triethoxy-2-methyl-butan als wasserklare Flüssigkeit mit einem Siedepunkt von 81-84°C und einem Gehalt nach GC von 98,2%.

### b) 1-Ethoxy-2-methyl-1,3-butadien

Dem im Beispiel 1b) beschriebenen Reaktor (gleiche Temperatur, gleicher Katalysator) wurden 60 ml/h 1,1,3-Triethoxy-2-methyl-butan und 20 l/h Stickstoff zugeführt. Vom eingesetzten 1,1,3-Triethoxy-2-methyl-butan wurden 90% umgesetzt; die Selektivität zu 1-Ethoxy-2-methyl-1,3-butadien betrug etwa 90%. Die Reaktionsprodukte wurden analog zu Beispiel 1 aufgearbeitet. Man erhielt 1-Ethoxy-2-methyl-1,3-butadien mit einer Reinheit von etwa 98%.

## Patentansprüche

1. Verfahren zur katalytischen Dealkoxylierung eines 1,1,3-Trialkoxy-2-methyl-butans der allgemeinen Formel
CH₃-CH(OR¹)-CH(CH₃)-CH(OR¹)₂ VI
worin R¹ C₁₋₄-Alkyl bedeutet,
zum entsprechenden 1-Alkoxy-2-methyl-1,3-butadien der allgemeinen Formel
H₂C=CH-C(CH₃)=CH-OR¹ II
in der Gasphase, **dadurch gekennzeichnet, dass** man als Katalysator schwach saures Aluminiumsilikat spezifischer Oberfläche im Bereich von 5 bis 50 m²/g verwendet.

2. Verfahren nach Anspruch 1, **dadurch gekennzeichnet, dass** der Aluminiumoxid-Anteil des Aluminiumsilikat-Katalysators in der α-Form vorliegt.

3. Verfahren-nach Anspruch 1 oder 2, **dadurch gekennzeichnet, dass** die katalytische Dealkoxylierung bei Temperaturen im Bereich von 250°C bis 400°C, vorzugsweise im Bereich von 300°C bis 350°C durchgeführt wird.

4. Verfahren nach einem der Ansprüche 1 bis 3, **dadurch gekennzeichnet, dass** die katalytische Dealkoxylierung bei Drücken von 1 kPa bis 200 kPa, vorzugsweise bei Atmosphärendruck, durchgeführt wird.

5. Verfahren nach einem der Ansprüche 1 bis 4, **dadurch gekennzeichnet, dass** die katalytische Dealkoxylierung in einem Festbettreaktor erfolgt, wobei das Edukt 1,1,3-Trialkoxy-2-methyl-butan kontinuierlich über den Aluminiumsilikat-Katalysator als Festbett in einer Reaktionssäule geleitet wird und das resultierende entsprechende 1-Alkoxy-2-methyl-1,3-butadien am Ende der Reaktionssäule entnommen wird.

6. Verfahren nach Anspruch 5, **dadurch gekennzeichnet, dass** das Edukt 1,1,3-Trialkoxy-2-methyl-butan mit einem Inertgas, beispielsweise Stickstoff oder Kohlendioxid, verdünnt ist.

7. Verfahren nach Anspruch 5 oder 6, **dadurch gekennzeichnet, dass** die Verweilzeit am Katalysatorbett im Bereich von 0,1 bis 10 Sekunden, vorzugsweise in Bereich von 0,5 bis 5 Sekunden, liegt.

## Claims

1. A process for the catalytic dealkoxylation of a 1,1,3-trialkoxy-2-methyl-butane of the general formula
CH₃-CH(OR¹)-CH(CH₃)-CH(OR¹)₂
wherein R¹ signifies C₁₋₄-alkoxy,
to the corresponding 1-alkoxy-2-methyl-1,3-butadiene of the general formula
H₂C=CH-C(CH₃)=CH-OR¹ II
in the gas phase, **characterized by** using weakly acid aluminium silicate of specific surface in the range from 5 to 50 m²/g as the catalyst.

2. A process according to claim 10, **characterized in that** the aluminium oxide content of the aluminium silicate catalyst is in the α-form.

3. A process according to claim 1 or 2, **characterized in that** the catalytic dealkoxylation is carried out at temperatures in the range from 250°C to 400°C, preferably in the range from 300°C to 350°C.

4. A process according to any one of claims 1 to 3, **characterized in that** the catalytic dealkoxylation is carried out at pressures from 1 kPa to 200 kPa, preferably at atmospheric pressure.

5. A process according to any one of claims 1 to 4, **characterized in that** the catalytic dealkoxylation is effected in a solid bed reactor, whereby the educt 1,1,3-trialkoxy-2-methyl-butane is continuously passed over the aluminium silicate catalyst as the solid bed in a reaction column, and the resulting corresponding 1-alkoxy-2-methyl-1,3-butadiene is removed at the end of the reaction column.

6. A process according to claim 5, **characterized in that** the educt 1,1,3-trialkoxy-2-methyl-butane is diluted with an inert gas, for example nitrogen or carbon dioxide.

7. A process according to claim 5 or 6, **characterized in that** the residence time on the catalyst bed is in the range from 0.1 to 10 seconds, preferably in the range from 0.5 to 5 seconds.

## Revendications

1. Procédé de désalcoxylation catalytique en phase gazeuse d'un 1,1,3-trialcoxy-2-méthylbutane de formule générale
CH₃-CH (OR¹)-CH(CH₃)-CH (OR¹)₂ (VI)
dans laquelle R¹ est un groupe alkyle en C₁₋₄,
pour donner le 1-alcoxy-2-méthyl-1,3-butadiène correspondant de formule générale
H₂C=CH-C(CH₃)=CH-OR¹ (II)
**caractérisé en ce qu'**on utilise en tant que catalyseur un silicate d'aluminium faiblement acide ayant une aire spécifique comprise dans la plage de 5 à 50 m²/g.

2. Procédé selon la revendication 1, **caractérisé en ce que** la partie oxyde d'aluminium du catalyseur à base d'un silicate d'aluminium se présente sous forme α.

3. Procédé selon la revendication 1 ou 2, **caractérisé en ce que** la désalcoxylation catalytique est mise en oeuvre à des températures comprises dans la plage de 250 à 400°C et de préférence dans la plage de 300 à 350°C.

4. Procédé selon l'une des revendications 1 à 3, **caractérisé en ce que** la désalcoxylation catalytique est mise en oeuvre sous des pressions de 1 à 200 kPa, de préférence sous la pression atmosphérique.

5. Procédé selon l'une des revendications 1 à 4, **caractérisé en ce que** la désalcoxylation catalytique a lieu dans un réacteur à lit fixe, la matière de charge 1,1,3-trialcoxy-2-méthylbutane étant envoyée en continu sur le catalyseur à base de silicate d'aluminium sous forme d'un lit fixe dans une colonne de réaction, et le l-alcoxy-2-méthyl-1,3-butadiène correspondant obtenu est soutiré à l'extrémité de la colonne de réaction.

6. Procédé selon la revendication 5, **caractérisé en ce que** la matière de charge 1,1,3-trialcoxy-2-méthylbutane est diluée avec un gaz inerte, par exemple l'azote ou le dioxyde de carbone.

7. Procédé selon la revendication 5 ou 6, **caractérisé en ce que** le temps de séjour dans le lit de catalyseur est compris dans la plage de 0,1 à 10 secondes et de préférence dans la plage de 0,5 à 5 secondes.
